# EUROPÄISCHE PATENTSCHRIFT

(11) **EP 1 425 018 B1**
(45) Veröffentlichungstag und Bekanntmachung des Hinweises auf die Patenterteilung: **22.10.2008**
(21) Anmeldenummer: 02796213.3
(22) Anmeldetag: 09.08.2002
(51) Int. Cl.: A61K 31/55, A61K 31/395, A61P 3/06

(54) **KOMBINATIONSPRÄPARATE VON 1,4- BENZOTHIEPIN-1,1-DIOXIDDERIVATEN MIT WEITEREN WIRKSTOFFEN UND DEREN VERWENDUNG**
COMBINED PREPARATIONS, CONTAINING 1,4-BENZOTHIEPINE-1,1-DIOXIDE DERIVATIVES AND OTHER ACTIVE SUBSTANCES, AND THE USE THEREOF
PREPARATIONS COMBINEES CONTENANT DES DERIVES DE 1,4-BENZOTHIEPINE-1,1-DIOXYDE AINSI QUE D'AUTRES SUBSTANCES ACTIVES ET UTILISATION DESDITES PREPARATIONS

(30) Priorität: 22.08.2001 DE 10140169; 31.08.2001 DE 10142456
(43) Veröffentlichungstag der Anmeldung: 09.06.2004
(73) Patentinhaber: Sanofi-Aventis Deutschland GmbH, 65929 Frankfurt am Main (DE)
(72) Erfinder: GLOMBIK, Heiner, 65719 Hofheim (DE); FRICK, Wendelin, 65510 Hünstetten-Beuerbach (DE); SCHAEFER Hans-Ludwig, 65239 Hochheim (DE); KRAMER, Werner, 55130 Mainz-Laubenheim (DE)
(86) Internationale Anmeldenummer: PCT/EP2002/008908
(87) Internationale Veröffentlichungsnummer: WO 2003/018024

(56) Entgegenhaltungen:
- WO-A-00/38725
- WO-A-02/50068
- WO-A-99/64409
- WO-A-99/64410
- US-B1- 6 221 897

## Beschreibung

Es sind bereits 1,4-Benzothiepin-1,1-dioxidderivate sowie deren Verwendung zur Behandlung von Hyperlipidämie sowie Arteriosklerose und Hypercholesterinämie beschrieben worden [vgl. US 6,221,897 oder WO 0038725].

Der Erfindung lag die Aufgabe zugrunde, Stoffgemische bzw. Kombinationspräparate von 1,4-Benzothiepin-1,1-dioxidderivaten mit weiteren Wirkstoffen zur Verfügung zu stellen, die eine synergistische Wirkung entfalten. Insbesonders sollte die hypolipidämische Wirkung der 1,4-Benzothiepin-1,1-dioxidderivaten in den Kombinationspräparaten durch die synergistische Wirkung mit weiteren Wirkstoffen überproportional gesteigert werden.

Die Erfindung betrifft daher ein Stoffgemisch enthaltend Verbindung der Formel V1, 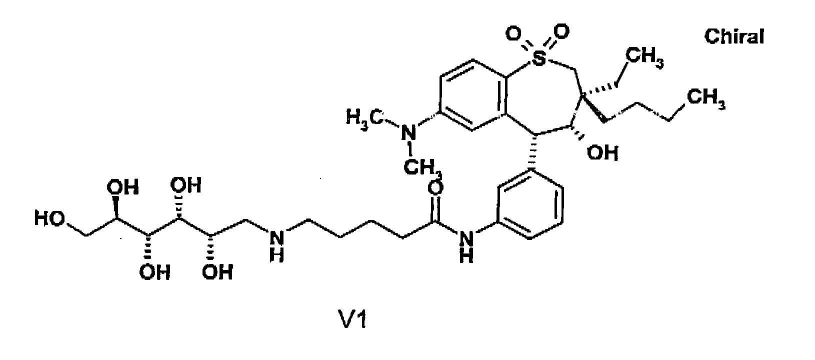 deren pharmazeutisch verträgliche Salze und Ezetimibe.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bemstein-, p-Toluolsulfon-, Wein- und Trifluoressigsäure. Für medizinische Zwecke wird in besonders bevorzugte Weise das Chloridsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Die Verbindungen der Formel I können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (V1)" auf Verbindung(en) der Formel (V1) wie vorstehend beschrieben, sowie ihre Salze und Solvate wie hierin beschrieben.

Die Menge einer Verbindung gemäß Formel (VI) sowie von weiteren Wirkstoffen, die erforderlich sind, um mit der Kombination den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg bis 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Aminopropanol-Ions. Vorzugsweise liegen die Stoffgemische jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit den Verbindungen als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (V1) abhängig ist. Auch dragierte Formulierungen und dragierte Retardformulierungen gehören in den Rahmen der Erfindung. Bevorzugt sind säure-und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.
Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (V1) sowie des weiteren Wirkstoffs enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (V1) sowie den weiteren Wirkstoff mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Die Verbindung der Formel (VI) wird in Kombination mit dem Cholesterinresorptionsinhibitor. Ezetimibe, verabreicht.

Es versteht sich, dass jede geeignete Kombination der erfindungsgemäßen Verbindungen mit einer oder mehreren weiteren pharmakologisch wirksamen Substanzen als unter den Schutzbereich der vorliegenden Erfindung fallend angesehen wird.

Die Kombinationspräparate bzw. Stoffgemische der Verbindungen der Formel (V1) stellen ideale Arzneimittel zur Behandlung von Lipidstoffwechselstörungen,und / oder Kohlenhydratstoffwechselstörungen dar, insbesondere von Hyperlipidämie und Metabolischem Syndrom. Die Kombinationspräparate eignen sich ebenfalls zur Beeinflussung des Serumcholesterinspiegels sowie zur Prävention und Behandlung arteriosklerotischer Erscheinungen.

Die folgenden Zubereitungen dienen zur Erläuterung der Erfindung ohne diese jedoch einzuschränken.

### Beispiel A

| Gelatineweichkapseln, enthaltend 100 mg Wirkstoffe pro Kapsel: | |
|---|---|
| | pro Kapsel |
| Wirkstoffe | 100 mg |
| aus Kokosfett fraktioniertes | |
| Triglycerid-Gemisch | 400 mg |
| Kapselinhalt | 500 mg |

### Beispiel B

| Emulsion, enthaltend 60 mg Wirkstoffe pro 5 ml: | |
|---|---|
| | pro 100 ml Emulsion |
| Wirkstoffe | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

### Beispiel C

| Rektale Arzneiform, enthaltend 40 mg Wirkstoffe pro Suppositorium: | |
|---|---|
| | pro Suppositorium |
| Wirkstoffe | 40 mg |
| Suppositoriengrundmasse | ad 2 g |

### Beispiel D

| Tabletten, enthaltend 40 mg Wirkstoffe pro Tablette: | |
|---|---|
| | pro Tablette |
| Laktose | 600 mg |
| Maisstärke | 300 mg |
| lösliche Stärke | 20 mg |
| Magnesiumstearat | 40 mg |
| | 1000 mg |

### Beispiel E

| Dragee, enthaltend 50 mg Wirkstoffe pro Dragees: | |
|---|---|
| | pro Dragee |
| Wirkstoffe | 50 mg |
| Maisstärke | 100 mg |
| Laktose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

### Beispiel F

Für die Herstellung des Inhalts von Hartgelatinekapseln eignen sich die folgenden Rezepturen:

| | | |
|---|---|---|
| a) | Wirkstoffe | 100 mg |
| | Maisstärke | 300 mg |
| | | 400 mg |
| | | |
| b) | Wirkstoffe | 140 mg |
| | Milchzucker | 180 mg |
| | Maisstärke | 180 mg |
| | | 500 mg |

### Beispiel G

| Tropfen können nach folgender Rezeptur hergestellt werden (100 mg Wirkstoff in 1 ml = 20 Tropfen): | |
|---|---|
| Wirkstoffe | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

Die synergistische Wirkung der Kombinationen der Verbindungen der Formel (V1) mit Ezetimibe wurde im Tierversuch getestet.

Die biologische Prüfung der erfindungsgemäßen Kombinationspräparate erfolgte am Hamster.

Für den Versuch wurden männliche Syrische Hamster (Mesocricetus auratus) im Alter von 8 bis 10 Wochen verwendet. Die Tiere erhielten eine mit 0.1 % Cholesterin angereichertes Standardfutter (Fa Teklad 8604M). Eine zusätzliche Normalkontrolle Gruppe erhielt nur Standardfutter.

Die Prüfsubstanzen wurden an 10 aufeinander folgenden Tagen 1 mal täglich oral mit einer Schlundsonde behandelt, die Kontrollgruppe wurde mit dem Vehikel behandelt.

Am Versuchstag 5 und 6 wurde der Kot zur Gallensäure-Analyse gesammelt. Am Versuchstag 10 wurde den Tieren retroorbital Blut entnommen und die Lipidspiegel im Plasma bestimmt. Am Versuchtag 9 wurden den Tieren oral radioaktive Tracer Appliziert, zur Bestimmung der Cholesterin Absorption anlog der von Zilversmith et al. beschriebenen Methode. Am Versuchstag 11 wurden die Tiere getötet, die Tiere Lebern wurden zur Cholesterin Analyse und zur Mikrosomen Präparation entnommen. In den Lebermikrosomen wurde ex vivo die Aktivität der 7 α-Hydroxylase bestimmt, nach eine modifizierten Methode von Hylemon et al.

Einfluß von Ezetimibe in Kombination mit V1 auf die Cholesterolresorbtion Versuchsdauer: 10 Tage

| | | | | |
|---|---|---|---|---|
| 1 | Teklad | | Normal Ktr. | n= 5 |
| 2 | Teklad | + 0,1% CH | Cholesterol Ktr. | n= 5 |
| 3 | Teklad | + 0,1% CH | 0,1mg/kg/d Ezetimibe (K0004513) | n= 5 |
| 4 | Teklad | + 0,1% CH | 0,1mg/kg/d V1 | n= 5 |
| 5 | Teklad | + 0,1% CH | 0,3mg/kg/d V1 | n= 5 |
| 6 | Teklad | + 0,1 % CH | 1 mg/kg/d V1 | n= 5 |
| 7 | Teklad | + 0, 1 % CH | 0,1mg/kg/d V1 + 0,1mg/kg/d Ezetimibe | n= 5 |
| 8 | Teklad | + 0,1% CH | 0,3mg/kg/d V1+ 0,1mg/kg/d Ezetimibe | n= 5 |
| 9 | Teklad | + 0,1% CH | 1mg/kg/d V1+ 0,1mg/kg/d Ezetimibe | n= 5 |

| | | | | |
|---|---|---|---|---|
| K00 04513 als Stammlsg. (1mg/ml in ETOH) eingesetzt Substanzen werden in 2% ETOH in 5% Enkonzentration gelöst. Anschließend werden die Lösungen mit 0,4% Kartoffelstärke suspendiert. Die Applikation erfolgt zwischen 7 - 8Uhr mit 10ml/kg **Futter:** Teklad 8604M CH: 032201 M **Versuchstiere:** Männlich Syrische Hamster (Mesocricetus auratus) Fa. Harlan 100-120 g zu Adaptionsbeginn **Meßparameter:** Futterverbrauch Tiergewicht (wöchentlich) Lebergewicht Safety-Parameter (CH; TG; ALAT/ASAT; AP; CK; HDULDL-CH) Lebercholesterol (HPLC) = 1x500mg in ETOH/KOH CYP7-Aktivität ( Lebermikrosomen als Gruppenpool je 0,5g - Präparation am Versuchstag) Kotsammlung am Tag 5-7 für Gallensäurebestimmung | | | | |

### Cholesterolsynthese:

### i.v. Applikation von ¹⁴C-Octanoat 10µCi/100g Tier 1 h vor Versuchsende (Isofluran-Narkose)

Entnahme von 2x 500mg Leber in EtOH/KOH

**Tabelle I:**

| | | **Plasma-Parameter** | | | | | | |
|---|---|---|---|---|---|---|---|---|
| Gruppe | Futter/Präparat | Cholesterin | | | Triglyceride | | | LDL |
| | | mmol/L | STABW | % | mmol/L | STABW | % | mmol/L |
| 1 | Normal Ktr. | 2,84 | ±0,09 | 81 | 2,01 | ±0,23 | 124 | 0,60 |
| 2 | Cholesterol Ktr. + 0,1% CH | 3,50 | ±0,27 | 100 | 1,62 | ±0,54 | 100 | 1,12 |
| 3 | +0.1% CH 0,1mg/kg/d Ezetimibe (K0004513) | 3,44 | ±0,64 | 98 | 1,63 | ±0.36 | 100 | 1,04 |
| 4 | + 0,1% CH 0,1mg/kg/d V1 | 4,20 | ±1.46 | 120 | 1,87 | ±0.30 | 115 | 1,06 |
| 5 | +0,1% CH 0,3 mg/kg/d V1 | 4,01 | ±0,89 | 114 | 1,75 | ±0,23 | 108 | 1,18 |
| 6 | +0,1% CH 1 mg/kg/d V1 | 3,23 | ±0,19 | 92 | 2,02 | ±0,57 | 124 | 0,92 |
| 7 | +0,1% CH 0,1 mg/kg/d V1 + 0,1 mg/kg/d Ezetimibe | 3,62 | ±0,18 | 103 | 2,08 | ±0,12 | 128 | 1,04 |
| 8 | + 0,1% CH 0,3 mg/kg/d V1 + 0,1 mg/kg/d Ezetimibe | 3,56 | ±0,94 | 101 | 2,11 | ±0,58 | 130 | 0,99 |
| 9 | + 0,1%CH 1 mg/kg/d V1 + 0,1 mg/kg/d Ezetimibe | 2,82 | ±0,05 | 81 | 1,84 | ±0,23 | 113 | 0,76 |

| | | | | | | | | |
|---|---|---|---|---|---|---|---|---|
| **Cholesterolresorption** p.o. Applikation von 2µCi ³H-Sitosterol/1µCi ¹⁴C-Cholesterol in 0,5ml 1:1 Tricaprin:Tricaprylin Kotsammlung von Tag 8 - 10 Der Kot wird anschließend getrocknet und zur lsotopenbestimmung im Oximat (Packard) verbrannt Aus der Tabelle ist abzulesen, daß die Verbindungen der Formel I in Kombination mit Ezetimibe einen synergistischen Effekt auf die Plasmaparameter zeigen. | | | | | | | | |

## Patentansprüche

1. Stoffgemisch enthaltend Verbindungen der Formel V1, 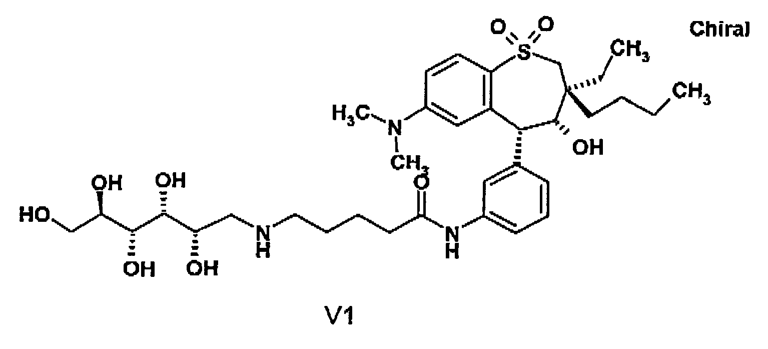 deren pharmazeutisch verträgliche Salze und Ezetimibe.

## Claims

1. A composition of matter comprising compounds of the formula C1, 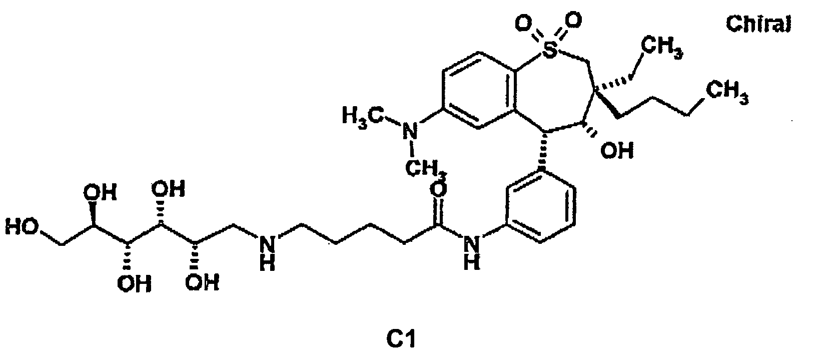 the pharmaceutically acceptable salts thereof, and ezetimibe.

## Revendications

1. Mélange de substances comprenant des composés de formule V1, 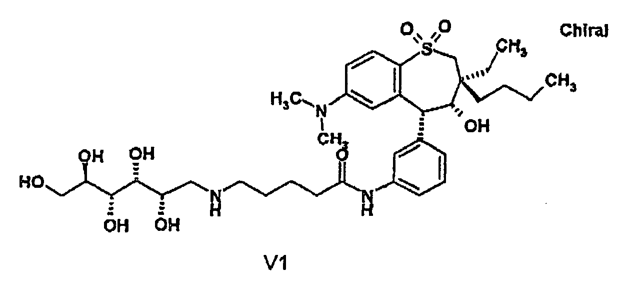 leurs sels pharmaceutiquement acceptables et des ézétimibes.
